# EUROPEAN PATENT APPLICATION

(11) **EP 1 527 755 A1**
(43) Date of publication of application: **04.05.2005**
(21) Application number: 05002606.1
(22) Date of filing: 06.04.1998
(51) Int. Cl.: A61F 2/06

(54) **Bifurcated stent with flexible side portion**

(30) Priority: 23.09.1997 US 935868; 12.01.1998 US 5988
(62) Divisional of application: 98912638.8
(71) Applicant: Vonderwalde Freidberg, Carlos, Colonia Roma, Mexico, D.F. 06700 (MX)
(72) Inventor: Vonderwalde Freidberg, Carlos, Colonia Roma, Mexico, D.F. 06700 (MX)
(74) Representative: Barz, Peter

(57) **Abstract**

A bifurcated stent comprises a main stent portion having a tubular body with first and second open ends, an interior lumen extending between the first and second ends, an outer diameter and a side opening located on the tubular body; and
a flexible side stent portion which has a tubular body with an outer diameter substantially smaller than the outer diameter of the tubular body of said main stent portion and with a first open end and a second open end,
wherein said side stent portion is secured by a first open end thereof on a perimeter of said side opening of said main stent portion with at least one connecting member defining an articulation so that said side stent portion is able to freely move at the articulation relative to said main stent portion.

## Description

### FIELD OF THE INVENTION

This invention relates to the field of expandable intraluminal support devices and more particularly to stents for treating lesions of arterial bifurcations and delivery systems therefor.

### BACKGROUND OF THE INVENTION

Typically, stents are expandable, tubular metallic devices that are positioned within a patient's vasculature and expanded in order to support a vessel and allow the flow of blood. Stents may be used to repair compromised blood vessels, such as those affected by obstructive coronary artery disease. Often, the stents are formed from a deformable metal and delivered using a balloon-type catheter. By advancing the catheter through a patient's vasculature, the stent may be delivered to a desired position. Inflating the balloon then deforms the stent into an expanded configuration, seating it within the artery.

However, in situations where the atherosclerotic lesion is located at a bifurcation of the vessel, stents pose certain problems. Most significantly, prior art stents do not offer complete coverage of the lesion site. As a result, smooth muscle cells can proliferate and migrate through gaps in the coverage, leading to restenosis.

Delivering a stent to bifurcated lesions also poses problems. One prior art technique involves placing a primary stent across the bifurcation and then introducing a secondary stent into the branch. However, this requires advancing first a guidewire and then a balloon catheter through the primary stent's framework to open a passage for the secondary stent. Neither procedure can be performed with certainty. The problems associated with stenting arterial bifurcations are exacerbated in the coronary anatomy due to size constraints. The coronary anatomy has a number of sites that commonly exhibit lesions in bifurcated areas, including the left anterior descending artery with diagonal branches, the circumflex artery with obtuse marginals, and others.

Thus, there remains a need for stents which offer more complete coverage of a lesion or lesions in bifurcated arterial locations, particularly for use in coronary arteries. This invention satisfies these and other needs.

### SUMMARY OF THE INVENTION

The invention is directed to an articulated bifurcated stent which can be easily mounted onto a delivery system while having sufficient flexibility and a low profile for delivery and a delivery system for the bifurcated stent.

According to the teachings of the present invention there is provided a bifurcated stent comprising a main stent portion having a tubular body with first and second open ends, an interior lumen extending between the first and second ends, an outer diameter and a side opening located on the tubular body; and a flexible side stent portion which has a tubular body with an outer diameter substantially smaller than the outer diameter of the tubular body of the main stent portion and with a first open end and a second open end, wherein the side stent portion is secured by a first open end thereof on a perimeter of the side opening of the main stent portion with at least one connecting member defining an articulation so that the side stent portion is able to freely move at the articulation relative to the main stent portion, preferably so that during the free movement there is substantially no deformation or buckling of neither the main stent portion nor of the side stent portion. According to a feature of the present invention, the connecting member or members substantially form a joint between the main stent portion and the side stent portion.

In an embodiment of the present invention, the side stent portion is secured by a first open end thereof on a perimeter of the side opening of the main stent portion at more than one point with at least two connecting members.

In an embodiment of the present invention, the connecting elements are sutures.

The bifurcated stent of the invention for treating a patient's bifurcated body lumen, such as coronary arteries, comprising a main stent portion having a length of about 10 to about 40 mm and a side portion having an end butted to the main portion so that the interior of the side stent portion is in fluid communication with the interior of the main stent portion through an opening. Preferably, one end of the side stent portion is secured to the main stent portion around the perimeter of the end of the side portion. The end is preferably secured with multiple sutures or other suitable means. The transverse dimensions of the side portion of the stent are generally substantially smaller, e.g. at least 10% smaller, preferably at least 20% smaller, than the transverse dimensions of the main portion of the stent. The passageway through the main stent portion is preferably of uniform and constant dimensions along the length thereof. For most vascular uses, the main stent portion is preferably expandable to about 2 to about 6 mm, most preferably about 2.5 to about 5 mm and the side stent portion is preferably expandable to about 1.5 to about 4 mm, most preferably about 2 to about 3.5 mm. Other body lumens may require greater or lesser expansions.

The bifurcated stent of the invention may be deployed in a bifurcated body lumen, such as in the patient's coronary anatomy, having a primary branch and a secondary branch by advancing a first and second guide wire into the primary and secondary branches, respectively. The side portion of the stent should be flexible enough or otherwise articulatied or hinged so as to readily fit into a secondary branch of the patient's bifurcated body lumen which may be at a variety of angles with respect to the primary branch of the patient's body lumen. The bifurcated stent is mounted on first and second catheters with the first catheter extending through the length of the main stent portion and the second catheter extending through part of the main portion, the opening in the wall of the main portion and into the side portion of the stent. The first and second catheters are then positioned over the first and second guidewires and intracorporeally advanced until the main portion of the stent is positioned at a desired location in the primary branch of the body lumen and the side portion of the stent is positioned at a desired location in the secondary branch of the body lumen.

The first catheter is similar to. and can be, an angioplasty catheter with an inflatable balloon on the distal extremity thereof. The second catheter may be the same as the first or may be a transfer catheter without a balloon on its distal extremity. If the first and second catheters are balloon catheters, the inflatable members are preferably inflated simultaneously to expand the main and side portions of the stent, but they may be inflated sequentially to sequentially expand these portions. If the second catheter is a transfer catheter without a balloon, the inflatable member of the first catheter is expanded to expand the main portion of the stent to anchor this portion of the stent within the main branch of the bifurcated artery and then is deflated and removed leaving the first guidewire in place. The second catheter without a balloon is withdrawn over the second guidewire and a catheter with a balloon is advanced over the second guidewire until the balloon on the second catheter replacement catheter is disposed within the interior of the side portion of the stent. The balloon on the replacement catheter is expanded to expand and secure the side portion of the stent within the arterial passageway. Once the main and side portions of the stent are expanded in the desired locations, the guidewires may be withdrawn. If desired, the bifurcated stent may be covered with a removable protective sheath after mounting the stent onto the expandable members of the first and second catheters, advanced within the patient's vasculature with the sheath in place and then remove the sheath before advancing the stent mounted catheters into the bifurcated region of the body lumen.

The present invention provides a simple stent system for bifurcated body lumens which is readily advanced within the patient and positioned within a bifurcated region of the patient's body lumen. These and other advantages will become more apparent from the following detailed description of the invention when taken in conjunction with the accompanying exemplary drawings.

### BRIEF DESCRIPTION OF THE DRAWINGS

Fig. 1 is an isometric view of the bifurcated stent of the invention.
Fig. 2 shows in part a method for creating and mounting the bifurcated stent onto a delivery system.
Fig. 3 is a transverse cross-sectional view of the system shown in Fig. 2 taken along the lines 3-3.

### DETAILED DESCRIPTION OF THE INVENTION

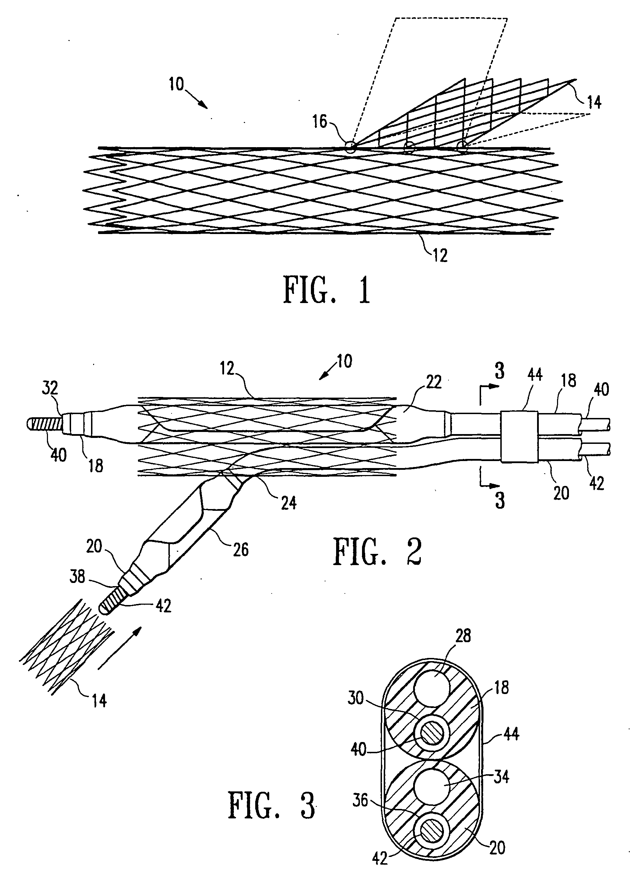

As shown in Fig. 1, a bifurcated stent 10 of this invention generally comprises a main portion 12 configured for use in the main branch of a bifurcated artery and a side portion 14 having an end butted to the main portion 12. The interior of side portion 14 opens into the interior of main portion 12. The main portion should have a length of about 10 mm to 40 mm, while the side portion 14 should have a length of about 6 mm to 8 mm.

Conventionally available metallic stents may be used with this invention. In particular, mesh or slotted tube designs are suitable. Preferred embodiments include the Micro Stent II or the GFX, available from Arterial Vascular Engineering; and the Multi-Link, available from Guidant. Of these, the Multi-Link is presently preferred as it presents a lower profile. Currently, a bifurcated stent formed from Multi-Link stents may be delivered through a 10 Fr guiding catheter while one formed from Micro Stent II stents requires an 11 Fr guiding catheter. Other stents may include the Paimaz-Shatz stent from Johnson and Johnson, the Gianturco from Cook Incorporated and other commercially available stents. Important characteristics of stents for use with this invention are low profile and sufficient flexibility to conform to the bifurcated artery, yet strong enough to maintain vessel patency. Suitable metallic materials include stainless steel and pseudoelastic nickel-titanium alloy. The stent portions may be expandable by expanding means such as balloons or they may be self expanding.

One end of the side portion 14 is butt joined or otherwise secured to the length of the main portion 12 by securing the perimeter of the end of the side portion. In a preferred embodiment. the stent portions are secured together with 6-0 or 7-0 polypropylene non-absorbable sutures 16. Generally, second stent 14 is butted at an opening in the framework of first stent 12. However, it may also be desirable to remove or cut one or more struts of the main portion 12 to create a sufficiently large opening so that the interior of side portion 14 has unobstructed communication with the interior of main portion 12 when the stent is in an expanded configuration. Other means of securing second stent 14 to first stent 12 may be suitable, including soldering, welding, using adhesives or other mechanical connections. An important feature of the butted attachment between the two portions of the stent is attachment around the perimeter of the end of side portions 14 to ensure complete coverage of the lesion by bifurcated stent 10 when it is expanded in the patient's body lumen. Prior art use of two stents within the separate body lumens which formed the bifurcation suffered from incomplete coverage of the lesion, which allowed tissue to migrate and proliferate through the gap, leading to restenosis. However, the side portion 14 of the stent should be flexible enough or be configured to articulate so the side portion can be moved radially to readily fit within the side branch of the body lumen. Ideally, the side portion 14 of the stent should be able to rotate so as to be readily insertable into any side branch of a body lumen and to be capable of being held generally parallel to the main portion 12 to facilitate delivery. The side portion 14 of the stent should be able to rotate through an angle of at least 60°, preferably at least 90°, so as to be readily insertable into most side branches of the lumens found in humans. The distal part of the main stent portion should likewise have some flexibility to facilitate entry into body lumens having Y-shaped configurations. The side portion 14 of the stent may be formed or assembled at an angle with respect to the main shaft portion 12 as shown in Fig. 1. The rotation of the side portion 14 is shown in phantom in Fig. 1.

Figure 2 shows bifurcated stent 10 being assembled and loaded for delivery on first and second delivery catheters 18 and 20 respectively. The length of stent 12 is positioned coaxially over the inflatable balloon 22 of delivery catheter 18 which is depicted in a folded condition in Fig. 2. The distal end of catheter 20 extends coaxially through the proximal part of the main portion 12 and then exits through an opening 24 corresponding to the communication between the interiors of the main and side portions 12 and 14. The length of the side portion 14 is positioned coaxially over the inflatable balloon 26 on the distal extremity of second delivery catheter 20 which is also shown in a folded condition in Fig. 2. If the second catheter 20 was a transfer catheter it may not have a balloon 26 as shown. Side portion 14 may then be butt joined to main portion 12 with sutures 16 as shown in Fig. 1 or other suitable means.

Delivery catheters 18 and 20 may comprise any suitable balloon catheter configured for deployment of a stent. As shown in Fig. 3, the delivery catheter 18 has an inflation lumen 28 which extends to and is in fluid communication with the interior of the balloon 22 on the distal extremity of the delivery catheter 18 and guidewire lumen 30 which extends to and is in fluid communication with a port 32 in the distal end of the delivery catheter. The second delivery catheter 20, as shown, also has an inflation lumen 34 and a guidewire lumen 36 as shown in Fig. 3 with the inflation lumen 34 extending to and in fluid communication with the interior of the balloon 26 and the guidewire lumen 36 extending to and in fluid communication with the port 38 in the distal end of the second delivery catheter. A first guidewire 40 is shown slidably and rotatably disposed within the guidewire lumen 34 and a second guidewire 42 is shown slidably and rotatably disposed within the guidewire lumen 36. The second delivery catheter 20 may also be a transfer catheter such as the Transit, from Cordis; the Buchbinder Transfer Catheter, from Medtronic; the Tracker 18, from Target Therapeutics; or others. The catheters 18 and 20 may be secured together along their length by bands 44 or continuously so that they may be advanced together without tangling their guidewires. If desired, both catheters 18 and 20 may be formed as a single catheter. For most coronary artery applications, only a few sizes of balloons 22 on the first delivery catheter 18 will be needed, e.g. 2.5 mm, 3.0 mm, 3.5 mm and 4.0 mm, to expand and deploy the main section of the bifurcated stent and a few similar but usually smaller sizes of balloons 26 on the second delivery catheter, e.g. 2.0 mm. 2.5 mm, 3.0 mm and 3.5 mm, will be needed to expand and deploy the side portion 14 of the stent. For other body lumens different sized stents and balloons may be required for lumen patency. The delivery catheters 18 and 20 may be formed of conventional angioplasty or stent delivery catheter materials. For example, the catheter shafts may be formed of high density polyethylene, polyesters such as Hytrel® and the balloons may be formed of polyethylene, polyethylene terephthalate, nylon, block copolymers such as PEBAX® and the like.

Alternatively, the first and second delivery catheters 18 and 20 may be formed into a single delivery catheter with a single main shaft, e.g. a single extrusion with two separate inflation lumens and two separate guidewire lumens, but with two separate distal sections each with inflatable balloons. The individual inflation lumens would extend to and be in fluid communication with separate inflation balloons and the individual guidewire lumens would extend to the distal ends of the separate distal sections.

Implanting the bifurcated stent may generally follow conventional procedures. The bifurcated stent 10 is loaded as described above, such that the first delivery catheter 18 extends through main portion 12 and second delivery catheter 20 extends through a proximal part of the main portion 12 of the first stent 1 and through the proximal portion 14. First and second guidewires 40 and 42 are backloaded into delivery catheters 18 and 20, respectively, and the catheters and guidewires are percutaneously introduced into the patient's vasculature by means of Seldinger techniques using a guiding catheter into the patient's arterial system. When the distal end of the assembly is proximally adjacent to the bifurcated arterial site, the first guidewire 40 is advanced out the distal end of delivery catheter 18 into the vasculature under fluoroscopic observation until it extends through the primary branch of the desired bifurcated artery beyond the targeted lesion site. The second guidewire 42 is advanced out the distal end of the second delivery catheter 20 until it extends well into the secondary branch of the bifurcated artery beyond the lesion site. Then, the catheters 18 and 20 are advanced over the guidewires until bifurcated stent 10 is properly positioned with the main portion 12 within the primary arterial branch and the side portion 14 within the secondary arterial branch. The balloon 22 on delivery catheter 18 is inflated to expand the main stent portion 12, seating it within the main arterial branch. The balloon 22 is deflated and then removed. The balloon 26 on the second delivery catheter 20 is inflated to expand side stent portion 14 and seat it within the secondary branch. Both balloons 22 and 26 may be simultaneously or sequentially inflated. However, after expanding the stent portions the catheters and the guidewires may be removed.

A general description of the device of the present invention as well as a presently preferred embodiment of the present invention has been set forth above. One skilled in the art will recognize and be able to practice many changes in many aspects of the device described above.

## Claims

1. A bifurcated stent (10) comprising a main stent portion (12) having a tubular body with first and second open ends, an interior lumen extending between the first and second ends, an outer diameter and a side opening located on the tubular body; and
a flexible side stent portion (14) which has a tubular body with an outer diameter substantially smaller than the outer diameter of the tubular body of said main stent portion (12) and with a first open end and a second open end,
wherein said side stent portion (14) is secured by a first open end thereof on a perimeter of said side opening of said main stent portion (12) with at least one connecting member defining an articulation so that said side stent portion (14) is able to freely move at the articulation relative to said main stent portion (12).

2. The bifurcated stent of claims 1, wherein during said free movement there is substantially no deformation or buckling of neither said main stent portion (12) nor said side stent portion (14).

3. The bifurcated stent of claim 1 or 2 wherein said at least one connecting member forms a joint between said main stent portion (12) and said side stent portion (14).

4. The bifurcated stent of any of claims 1-3, wherein said side stent portion (14) is secured by a first open end thereof on a perimeter of said side opening of said main stent portion (12) at more than one point with at least two connecting members.

5. The bifurcated stent of any of claims 1-4 wherein said connecting elements are sutures.

6. The bifurcated stent of any of claims 1-5 wherein the said main stent portion (12) is expandable to a diameter of 2 to 6 mm, preferably 2.5 to 5 mm.

7. The bifurcated stent of any of claims 1-6 wherein said side stent portion (14) is expandable to a diameter at least 10% smaller than the diameter of said main stent portion (12).

8. The bifurcated stent of claim 7 wherein said side stent portion (14) is expandable to a diameter at least 20% smaller than the diameter of said main stent portion (12).

9. The bifurcated stent of any of claims 1-8 wherein said side stent portion (14) is configured to rotate through an angle of at least about 60° at said articulation.

10. The bifurcated stent of claim 9, wherein said side stent portion (14) is configured to rotate through an angle of at least about 90° at said articulation.
